# EUROPEAN PATENT APPLICATION

(11) **EP 4 455 126 A1**
(43) Date of publication of application: **30.10.2024**
(21) Application number: 23155540.0
(22) Date of filing: 08.02.2023
(51) Int. Cl.: C07C 317/10, C07C 317/14, C07C 317/22, C07C 317/24, C07C 291/04, C07C 315/02, C07F 9/66, C07C 391/02

(54) **LIGHT-MEDIATED AEROBIC OXIDATION METHOD**

(71) Applicant: Ecosynth, 9800 Deinze (BE); Van Aken, Koen Jeanne Alfons, 8520 Kuurne (BE)
(72) Inventor: Diprima, Damiano, 2018 Antwerpen (BE); Van Aken, Koen Jeanne Alfons, 8520 Kuurne (BE); Gemoets, Hannes, 4824EZ Breda (NL)
(74) Representative: Arnold & Siedsma

(57) **Abstract**

The present invention is to provide a system that can be used to oxidize a series of heteroatoms to their corresponding oxides in a very selective way, using (visible/UVA) light activation without the need of an extra photocatalyst and tolerates the use of water, optionally as a solvent. The method comprising the steps of: a) providing an organic compound comprising a heteroatom in a reaction medium comprising H₂O, and b) irradiating the reaction medium comprising the compound provided at step a) in the presence of a source of oxygen with light having a wavelength in the UV-vis electromagnetic spectrum, thereby oxidating said heteroatom. It further provides the use of said method in amongst others the treatment of waste-water streams.

## Description

### FIELD OF THE INVENTION

The present invention relates to the field of chemical technology, materials and life sciences, and specifically relates to a light-mediated aerobic oxidation method.

### BACKGROUND TO THE INVENTION

Oxidation is a fundamental reaction type in both research as in industrial production. An important subclass is the oxygenation of compounds containing heteroatoms. Typical examples are the selective oxidation of sulfides to high value sulfoxides or oxidative desulfurization in treatment processes.

Traditionally, oxidations require strong oxidants such as KMnO₄, Na₂Cr₂O₇, CH₃COOOH, Na₂S₂O₈ or KIO₄ which generally create many problems in storage, operation and post-processing. As usually water washing is required in this post-processing, large amount of waste streams (often containing toxic heavy metal ions) are generated which treatments substantially add to the overall operational costs.

In the search of more efficient oxidation methods, molecular oxygen has attracted significant attention as an alternative oxidant due to its low cost, abundancy, high atom economy and the generation of water as an environmental friendly waste product. However, oxygen still requires specific additives such as metal catalysts, ligands or biological enzymes in order to react. Often it needs need to be used in pure form or under extreme reaction conditions (high temperature or pressure) which poses safety risks, This limits its use in industrial settings.

Therefore, the use of aerobic oxidation methods which run under (close to) ambient temperature and pressure are important. In this respect, activation with light is particularly attractive. However, to initiate reactions in the visible light/UVA region, a photocatalyst is needed which brings not only extra cost but also adds to the purification efforts. In addition, the use of photocatalyst usually requires organic solvents.

Rajabi et al., 2021 describes light-mediated photocatalyzed reactions wherein sulfides are oxidated in an acqueous environment to sulfoxides at room temperature in the presence of a tungsten polysiloxane network.

In this respect, light-mediated reactions not requiring an extra photocatalyst are particularly attractive but scarcely described. For the oxidation of sulfides to sulphoxide only two examples are known.

Bonesi et al., 2017, describes irradiating sulfide compounds at wavelength lower than 310 nm results in a complex mixture containing sulphoxides and other components originating form a C-S cleavage.

Fan et al., 2021, describes a blue light-mediated (> 405 nm) system for the oxygenation of sulfides to selectively form sulphoxide without an extra photocatalyst. However, the reaction is very slow (over ten hours to reach full conversion), requires pure oxygen (the aerobic conditions yields only 10% conversion in 24 hours) and needs organic solvent (no reaction observed in water). The non-selectivity or slow reactions rates makes the above known examples of limited practical use for industrial chemical production.

Hence, there is a need for an oxidation method which is efficient, simple to implement, and which does not require a complex catalytic system.

### SUMMARY OF THE INVENTION

In a first aspect, the present invention relates to a method of oxidation of an organic compound comprising a heteroatom selected from S, Se, P, N, the method comprising the steps of:
a) providing an organic compound comprising a heteroatom selected from S, Se, P, N, in a reaction medium comprising H₂O, and optionally a reaction additive;
b) irradiating the reaction medium comprising the compound provided at step a) with light having a wavelength in the UV-vis electromagnetic spectrum, thereby oxidating said heteroatom, wherein
step b) is conducted in the presence of a source of oxygen. An advantage of the method according to the present invention is that it uses visible/UVA light activation without the need of an extra photocatalyst. Yet another advantage of the method of the present invention is that the method tolerates the use of water in the reaction medium, and optionally water as solvent.

According to an embodiment of the present invention, step b) is conducted in the presence of a compound comprising at least one aromatic moiety. The aromatic moiety can be for example be part of the organic compound, the reaction additive or and/or a solvent in the reaction medium.

It was surprisingly found that the presence of a compound comprising an aromatic moiety during step b) is advantageous in providing oxidation products of S, Se, P and N in a very selective way, and with high conversion yields.

This enables the green synthesis of oxidation products of heteroatoms in a wide range of substrates with high space time yields, low cost, high atom economy. It is simple and practical and requires minimal post processing.

According to yet another embodiment of the present invention, the aromatic moiety is a phenyl ring, substituted or unsubstituted. An advantage of the present embodiment is that phenyl rings have been found to provide for good yields both in additives as in oxidation substrates. Phenyl rings are also commonplace so that the oxidation does not require for exotic molecular structures to provide high yields.

According to yet another embodiment of the present invention, the reaction medium is H₂O. An advantage of the present embodiment is that the use of water in the reaction medium allows for an environmentally friendly oxidation allowing for high conversion and selectivity.

According to yet another embodiment of the present invention, at step a) the reaction medium further comprises a solvent, preferably a polar solvent, which polar solvent is preferably selected from the list comprising: acetonitrile, ethanol, methanol, tetrahydrofuran, 2-Methyltetrahydrofuran, acetone, gamma-Valerolactone, cyrene and the like, preferably acetonitrile. It was found that these solvents are particularly beneficial to be obtainable yields.

According to yet another embodiment of the present invention, the reaction medium comprises H₂O and acetonitrile in a ratio HzO:acetonitrile from 100:0 to 1:99, preferably 20:80. It was found that the present embodiment allows for really good conversion to the oxidation products.

According to yet another embodiment of the present invention, at step b) light is with a wavelength from 320nm, preferably, the light has a wavelength from 320nm to 400nm.

According to yet another embodiment of the present invention, at step a) the compound is provided as part of a water stream to be treated, such as a waste-water stream, such as a waste-water desulfurization treatment.

According to yet another embodiment of the present invention, steps a) and b) are carried out as a continuous flow oxidation. It was found that the method of the present invention is particularly suitable for continuous flow oxidation and benefits from the high-throughput and solvent reutilization possibilities provided by this system.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings is provided to make apparent to those skilled in the art how the several forms of the invention may be embodied in practice.

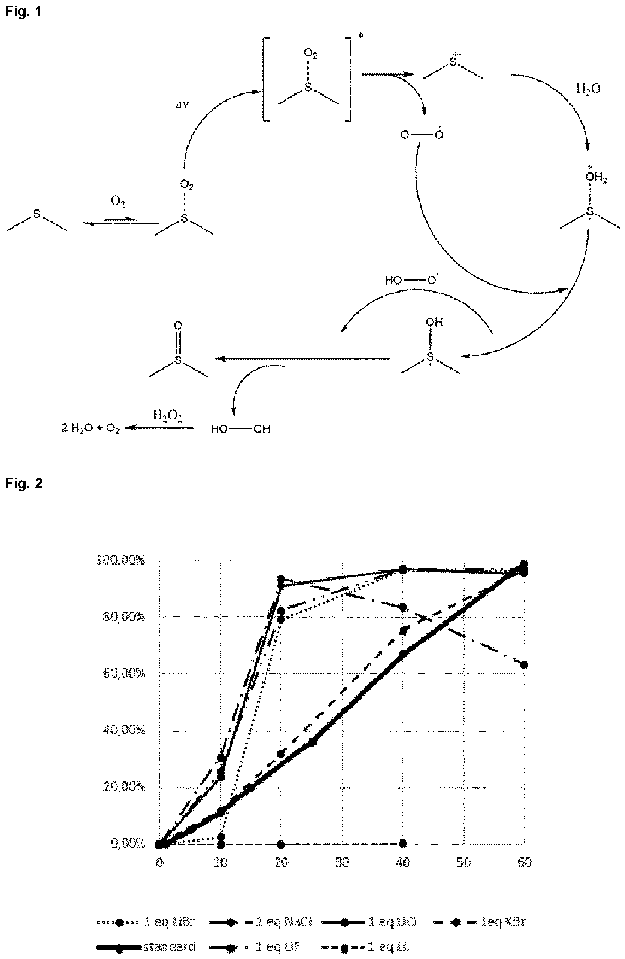

**Figure 1****,** also referred to as **Fig. 1****,** illustrates the possible reaction mechanism for the oxidation according to the present invention.
**Figure 2****,** also referred to as **Fig. 2****,** illustrates results of studies on the influence of a number of salts on a reaction carried out according to an embodiment of the present invention.
**Figure 3****,** also referred to as **Fig. 3****,** illustrates results of studies on the influence of some selected acids and bases on a reaction carried out according to an embodiment of the present invention.
**Figure 4****,** also referred to as **Fig. 4****,** illustrates a schematic overview of a flow reaction apparatus with which a method according to an embodiment of the present invention can be implemented.

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. When describing the compounds of the invention, the terms used are to be construed in accordance with the following definitions, unless a context dictates otherwise.

The term "about" or "approximately" as used herein when referring to a measurable value such as a parameter, an amount, a temporal duration, and the like, is meant to encompass variations of +/- 10 % or less, preferably +/- 5 % or less, more preferably +/- 1 % or less, and still more preferably +/- 0.1 % or less of and from the specified value, insofar such variations are appropriate to perform in the disclosed invention. It is to be understood that the value to which the modifier "about" or "approximately" refers is itself also specifically, and preferably, disclosed.

In the context of the present invention, "a/an", should eb interpreted as one or more. For example, with "an organic compound", reference should be made to one or more organic compounds.

By means of the term "waste-water stream", reference is made to a stream wherein water is the most abundant liquid medium, and where the stream is the result of a process wherein water has been used, such as sewage streams, wastewater streams and industry effluents.

By means of the term "UV-vis electromagnetic spectrum", reference is made to the region of the electromagnetic spectrum from about 190nm to about 900nm. According to yet another embodiment of the present invention, at step b) light is with a wavelength from 320nm. According to yet a further embodiment of the present invention, at step b) light is with a wavelength from 320nm to 400nm, preferably from 350nm to 390nm.

In a first aspect, the present invention relates to a method of oxidation of an organic compound comprising a heteroatom selected from S, Se, P, N, the method comprising the steps of:
a) providing an organic compound comprising a heteroatom selected from S, Se, P, N, in a reaction medium comprising H₂O, and optionally a reaction additive;
b) irradiating the reaction medium comprising the compound provided at step a) with light having a wavelength in the UV-vis electromagnetic spectrum, thereby oxidating said heteroatom, wherein
step b) is conducted in the presence of a source of oxygen. An advantage of the method according to the present invention is that it uses visible/UVA light activation without the need of an extra photocatalyst. Yet another advantage of the method of the present invention is that the method tolerates the use of water in the reaction medium, and optionally water as solvent.

According to an embodiment of the present invention, step b) is conducted in the presence of a compound comprising at least one aromatic moiety. It was surprisingly found that the presence of an aromatic moiety either as part of a reaction additive or as part of the organic compound provided at step a), allows for the oxidation of heteroatoms selected from S, Se, P, N. The method according to the present invention is advantageous in providing oxidation of S, Se, P and N in a very selective way, and by providing improved conversion yields.

The present invention allows to operate a commercially viable and important reaction in a safe and simple to operate matter with a very green profile. No side product are generally formed and no to little wastes can be generated. The reaction is very robust tolerating a variety of functional groups producing the desired product in high yield and with good atom economy making it ideal for some late stage functionalization. The process can be easily translated into a flow process essential step in order to scale up photocatalytic reactions and achieve great productivities

In light of the above, the method according to the present invention can be beneficially used in a variety of industrial processes, such as, and not limited to: the desulfurization of streams, functionalization of peptides through selective oxidation of methionine and cysteine, late-stage functionalization of APIs, synthesis of fine chemicals, and chemicals, selective functionalization of sulfur-contained polymers (e.g. Polyethylene sulfide)), preparation of sulfoxide or phosphine oxide containing molecules for coordination chemistry.

According to an embodiment of the present invention, the reaction additive and/or one or more compounds comprise at least one aromatic moiety, the aromatic moiety being part of the organic compound comprising the heteroatom provided at step a).

By means of the term "organic compound", reference is made to a compound wherein one or more atoms of carbon are covalently linked to atoms of other elements, most commonly hydrogen, oxygen, or nitrogen. According to the present invention, the organic compound being oxidized comprises at least one heteroatom selected from the elements sulphur (S), selenium (Se), phosphorous (P), and nitrogen (N).

By means of the term "reaction additive", reference is made to a chemical entity which improves the oxidation yield of the compound provided at step a). According to an embodiment of the present invention, the reaction additive is a compound comprising an aromatic moiety, a salt, an acid or combinations thereof.

By means of the term 'aromatic moiety' reference is made to a polyunsaturated, aromatic hydrocarbyl group having a single ring (i.e. phenyl) or multiple aromatic rings fused together (e.g. naphthalene or anthracene) or linked covalently, typically containing 6 to 10 atoms; wherein at least one ring is aromatic. The aromatic ring may optionally include one to three additional rings (either cycloalkyl, heterocyclyl, or heteroaryl) fused thereto. Aryl is also intended to include the partially hydrogenated derivatives of the carbocyclic systems enumerated herein. Non-limiting examples of aryl comprise phenyl, biphenylyl, biphenylenyl, 5- or 6-tetralinyl, 1-, 2-, 3-, 4-, 5-, 6-, 7-, or 8-azulenyl, 1- or 2-naphthyl, 1-, 2-, or 3-indenyl, 1-, 2-, or 9-anthryl, 1- 2-, 3-, 4-, or 5-acenaphtylenyl, 3-, 4-, or 5-acenaphtenyl, 1-, 2-, 3-, 4-, or 10-phenanthryl, 1- or 2-pentalenyl, 1, 2-, 3-, or 4-fluorenyl, 4- or 5-indanyl, 5-, 6-, 7-, or 8-tetrahydronaphthyl, 1,2,3,4-tetrahydronaphthyl, 1,4-dihydronaphthyl, dibenzo[a,d]cylcoheptenyl, and 1-, 2-, 3-, 4-, or 5-pyrenyl. The aromatic moiety can also comprise one or more heteroatoms, in other words, the aromatic moiety can be an heteroaromatic ring, such as a pyridine ring. According to an embodiment of the present invention, the aromatic moiety present during step b) is a phenyl ring, substituted or unsubstituted. It was found that the aromatic moiety is preferably a phenyl ring, substituted or unsubstituted.

In order to solve the above-mentioned problems, the object of the present invention is to provide a system that can be used to oxidize a series of heteroatoms to their corresponding oxides in a very selective way, using (visible/UVA) light activation without the need of an extra photocatalyst and tolerates the use of water, optionally as a solvent.

This enables the green synthesis of oxidation products of heteroatoms in a wide range of substrates with high space time yields, low cost, high oxygen atom economy. It is simple and practical and requires minimal post processing.

According to an embodiment of the present invention, the compound comprising the aromatic moiety is the organic compound comprising the heteroatom to oxidize. According to yet another embodiment of the present invention, the compound comprising the aromatic moiety is a reaction additive present during step b). According to yet another embodiment of the present invention, the compound comprising the aromatic moiety is a solvent or co-solvent present in the reaction medium.

The aromatic moiety can be selected from: phenyl, pyridine, naphthalene and the like, substituted or unsubstituted; more in particular wherein the aromatic moiety is a phenyl ring, substituted or unsubstituted.

The aromatic moiety according to the present invention can comprise substituting groups such as electro-withdrawing groups (EWG), such as -CF₃ and/or an electro-donating group (EDG), such as -NH₂. According to a specific preferred embodiment of the present invention, the aromatic moiety comprises an EDG group. According to yet another embodiment of the present invention the aromatic moiety is an electron-rich aromatic moiety.

By means of the term EDG group, reference is made to a group selected from, and not limited to: oxido group, (substituted) amino groups, hydroxy and alkoxy groups, acylamido groups, acyloxy groups, (di)alkylphosphino, alkylthio, and sulfhydryl groups, phenyl (or aryl) group, vinyl group, alkyl groups (e.g. -CH₃, -C₂H₅), carboxylate group.

It was found that electron-rich aromatic moieties, such as those in toluene and anisole, provide for improved conversion and selectivity compared to electron-deficient aromatic moieties, such as that in Trifluorotoluene.

Hence, at step a) the organic compound is provided in a reaction medium comprising water. The reaction medium can hence be water or a mixture comprising water and one or more other solvents, such as polar solvents, such as acetonitrile. It was found that the presence of water, even in minimal amounts, within the reaction medium, provides for higher conversion yields.

By means of the term "reaction medium", reference is made to a solution, mixture or compound which serves as environment for the oxidation reaction according to the present invention to take place. One of the benefits of the method according to the invention, is its ability to be performed in water (H₂O) as reaction medium. This allows use of the method on water treatment installations, with for example oxidation and/or destruction of malodors compounds such as Volatile Organic Sulfur Compounds (VOSCs). In accordance with an embodiment of the present invention, the reaction medium is H₂O. When the reaction medium is H₂O, the oxidation is more environmentally friendly.

Besides water the reaction medium may further comprise a solvent; in particular a polar solvent, such as for example selected from the list comprising: acetonitrile, ethanol, methanol ... and the like. Polar solvents were seen to provide improved conversion yields. In particular embodiment the aqueous reaction medium further comprises acetonitrile (ACCN) as the polar solvent. It was surprisingly found that the presence of water and acetonitrile in the reaction mixture allows for faster kinetics and or better selectivity towards the desired oxidation product. In the examples a H₂O:acetonitrile ratio of 20:80 has been used. Evidently other ratio's can be used as long a certain amount of water is actually present, so at least 1% should be present, solvent is not necessary so water content can go up to 100%. The preferred ratio is often determined by the solubility of the substrates. Expressed differently, in a particular embodiment the reaction medium comprises H₂O and a polar solvent, in particular acetonitrile in a ratio from 100:0 to 1:99, preferably H₂O:acetonitrile in a ratio of 20:80.

In step b) of the method according to invention a reaction additive is optionally present. Within the context of the present invention, the reaction additive is a compound preferably selected from the list comprising: toluene, benzene, xylene, alkali and earth alkali halides, salts reaction (e.g. but not limited to NaCl, LiCI, LiBr) other in the examples. In said instances wherein the organic compound comprising a heteroatom lacks an aromatic moiety, the reaction additive is a compound preferably selected from the list comprising: toluene, benzene, xylene and the like.

According to an embodiment of the present invention, the reaction medium comprises a reaction additive which is a salt selected from: LiBr, NaCl, LiCI, KBr, LiF. According to yet a further embodiment of the present invention, the reaction additive is a chloride, a fluoride or a bromide of an alkali metal or alkaline earth metal and preferably an alkali metal chloride (such as NaCl, LiCI) or alkaline earth metal chloride (such as MgCl₂), more preferably an alkali metal chloride, such as LiCI. It was found that the present embodiments provide for improved conversion yield and selectivity. According to yet a further preferred embodiment of the present invention, the reaction additive is LiCI. It was found that LiCI provide for a more robust oxidation process, so that even under prolongated irradiation time there is retention of the selectivity, and it is easier to separate after the oxidation reaction.

The organic compound comprising a heteroatom is hereinafter also referred to as the 'Substrate'. In an embodiment according to the method of the invention the heteroatom is S. In an embodiment according to the method of the invention the heteroatom is P. In an embodiment according to the method of the invention the heteroatom heteroatoms is Se. In an embodiment according to the method of the invention the heteroatom is N.

In an embodiment according to the invention, the substrate comprises a functional group selected from: R-S-R, R-S-S-R, R-S-H, R-Se-H, R-Se-R, R-Se-Se-R, P-R₃, P-R₂H, P-RH₂, As-R₃, As-RX₂, As-R₂X, N-Rs, N-R₂H, and N-RH₂, wherein X is a halogen and R is independently selected from an aromatic moiety or an alkyl moiety, substituted or unsubstituted.

The term "halo" or "halogen" as a group or part of a group is generic for fluoro, chloro, bromo, or iodo.

The term "alkyl" by itself or as part of another substituent refers to a fully saturated hydrocarbon of Formula CxH2x+1 wherein x is a number greater than or equal to 1. Generally, alkyl groups of this invention comprise from 1 to 20 carbon atoms. Alkyl groups may be linear or branched and may be substituted as indicated herein. When a subscript is used herein following a carbon atom, the subscript refers to the number of carbon atoms that the named group may contain. Thus, for example, C₁₋₄alkyl means an alkyl of one to four carbon atoms. Examples of alkyl groups are methyl, ethyl, n-propyl, i-propyl, butyl, and its isomers (e.g. n-butyl, i-butyl and t-butyl); pentyl and its isomers, hexyl and its isomers, heptyl and its isomers, octyl and its isomers, nonyl and its isomers; decyl and its isomers. C₁-C₆ alkyl includes all linear, branched, or cyclic alkyl groups with between 1 and 6 carbon atoms, and thus includes methyl, ethyl, n-propyl, i-propyl, butyl and its isomers (e.g. n-butyl, i-butyl and t-butyl); pentyl and its isomers, hexyl and its isomers, cyclopentyl, 2-, 3-, or 4-methylcyclopentyl, cyclopentylmethylene, and cyclohexyl.

The term "optionally substituted alkyl" refers to an alkyl group optionally substituted with one or more substituents (for example 1 to 4 substituents, for example 1, 2, 3, or 4 substituents or 1 to 2 substituents) at any available point of attachment. Non-limiting examples of such substituents include halo, hydroxyl, carbonyl, nitro, amino, oxime, imino, azido, hydrazino, cyano, aryl, heteroaryl, cycloalkyl, acyl, alkylamino, alkoxy, thiol, alkylthio, carboxylic acid, acylamino, alkyl esters, carbamate, thioamido, urea, sullfonamido and the like.

By means of the term "source of oxygen", reference is made to molecular oxygen or one or more of compounds which are provided to release molecular oxygen during the irradiation of the reaction medium with light, thereby oxidizing the organic compound at the location of said heteroatom selected from S, Se, P and N. According to yet another embodiment of the present invention, the source of oxygen is a mixture comprising nitrogen and oxygen, such as air. The use of air as source of oxygen is particularly beneficial in allowing the use of a readily available oxidant thereby reducing costs.

In principle any source of oxygen can be used, including a mixture comprising nitrogen and oxygen, such as air. The latter adds to the simplicity of the method according to the invention that can be performed in open system at atmospheric pressure. As evident from the examples, simply bubbling air through the reaction medium is sufficient to achieve the fast and complete conversion of the substrates. As such not only batch processing, but also continuous flow oxidation treatments are possible, such as for example continuous flow oxidation of a water stream or waste-water stream to be treated.

By means of the term "continuous flow oxidation", reference is made to an oxidation reaction carried out under a continuous flow. Continuous flow involves the use of channels or tubing to conduct said oxidation reaction, thereby reducing reaction times, higher selectivities, straightforward scalability, and the possibility to safely use hazardous intermediates and gaseous reactants. The continuous flow oxidation could be carried out in continuous flow microreactors. According to yet another embodiment of the present invention, steps a) and b) are carried out as a continuous flow oxidation. It was found that the method of oxidation accordance to the present invention is well suited to be implemented in continuous flow reaction systems. This was unexpected.

The choice of reactor materials and reactor types also depends on the operational conditions. Especially at high temperatures and elevated pressures, one should carefully select the reactor material in order to withstand these harsh conditions. Stainless steel microreactors can resist high temperature and high pressure, and provide a high heat conductivity. However, stainless steel is not compatible with acidic media except for those with special surface treatments. PFA, FEP or PTFE microreactors have the advantages of easy fabrication, low cost and high flexibility. They can resist strong acidic and alkaline media at low to moderate temperatures and pressures. Such polymeric materials are transparent which allows one to study the hydrodynamics inside the microchannel. This is beneficial for improving the reactor design and the overall process. A high transparency is also crucial for photochemical processes (e.g., singlet oxygen generation); PFA and FEP are transparent for both UV and visible light. Nevertheless, PFA microreactors cannot resist high temperatures and the limiting pressure decreases significantly with increasing temperatures. Importantly, these polymers have a low thermal conductivity, which can be a problem for highly exothermic reactions.

Silicon microreactors have similar transparency properties and better thermal conductivity than polymer-based reactors. It can withstand high temperatures and high pressures, and provide chemical resistance to a broad range of chemicals. However, they are not always resistant to strong alkaline reaction mixtures, especially at elevated temperatures. Surface treatments can help to overcome this limitation. Silicon carbide has an even higher thermal conductivity and combines it with an exceptional chemical stability. However, this material is quite expensive and is also brittle.

The method according to the present invention provides for high yields under (close to) ambient temperature, thereby allowing the use of microchannels which do not require to withstand medium to high temperatures.

The method according to the present invention is particularly useful in the water treatment installations, such as waste-water treatment installations, with for example oxidation and/or destruction of malodors compounds such as Volatile Organic Sulfur Compounds (VOSCs), i.e. for use in a desulfurization treatment. By means of the term "desulfurization treatment" or "desulphurisation treatment", reference is made to a chemical process for the removal of sulphur from an entity comprising said element. By means of the method of present invention, a variety of aqueous streams can be treated, such as, and not limited to: petroleum and coal (waste) streams containing sulphureous organic compounds by oxidation and removal, neutralization of chemical warfare as mustard gas, deodorization of wastes (industrial, water, household) from SH containing molecule like mercaptans.

In an embodiment according to the invention at step b) light having a wavelength in the UV-vis electromagnetic spectrum with a wavelength from 320 nm, in particular from about 365 nm is used.

### EXPERIMENTAL PART

The present experimental part shows how the present invention can be put into practice. In particu lar.

### MATERIALS AND METHODS

The reactions are performed in a 20 mL test tube. The test tube is placed on a stirring plate stirring vigorously the solution to maximize the gas liquid mixing. Oxygen or air is bubbled in the solution using a 1/16 ptfe tube from a balloon. The reaction tube is closed using a silicon septum and the gas leaves through a 1.2 mm needle. The reaction is irradiated using a Pheseon FireEdge FE400 240×10AC365-4W LED system placed at a distance between 0.2 and 5 cm from the reactor. The system is then cooled using a fan Sunon DP201A.

### MATERIALS

### SUBSTRATE SCOPE

The method according to the present invention was tried onto a variety of substrates and reaction conditions. In particular, the method of the present invention allows for oxidation of an organic compound comprising a heteroatom selected from S, Se, P, N. The substrates that could be oxidized in accordance with the method of the present invention include, and are not limited to: Thioanisole, Thiophenol, Diphenyl sulfide, Benzyl methyl sulfide, Tetrahydrothiophene, Diphenyl selenide, Benzylmercaptane, Triphenylphospine, Phenyl cyclopropyl sulfide, p-Hydroxymethylthioanisole, p-Acetylthioanisole, 4-Methoxythioanisole, Para methyl thioanisole, 2-(Methylthio)pyridine, 2-Methylthiobenzoxazole, p-lodothioanisole, p-Fluorothioanisole, p-Clorothioanisole, 3,5-dichloro thioanisole, 4-Hydroxytetrahydrothiopyran, Triisopropyl phosphite, Trimethyl phosphite, Bis(2-Diphenylphosphinophenyl)ether, Albendazole.

The oxidation of sulfides might lead to two products: Sulfoxides and Sulfone. Thus, for the following reactions the results will be given trough conversion and selectivity. Conversion is defined as the amount of starting material that is converted, whereas the selectivity is the amount of the converted material effectively producing the sulfoxide product. Similarly, conversion and selectivity are used for other experiments pertaining to oxidation reactions on different substrates in which more than one product is expected. In many experiments, thioanisole was used as test molecule to explore the reaction and therefore all the parameter have been primarily tested on it.

### - Oxidation of Thioanisole (1) to (2) - standard conditions

The reactant thioanisole (0.6mmol) was dissolved in a 5 mL volumetric flask using a mixture of CH₃CN (80%) and H₂O (20%). The solution is then transferred in a 20mL test tube and stirred with a magnetic stirrer. The reaction tube is irradiated using a violet LED (peak wavelength 365nm, Phoseon FE400 80W 365nm LED, Lamp is in close proximity of the tube at 0,5 cm), at room temperature, using air or oxygen atmosphere (1atm). Reaction is followed via GC FID diluting 100uL of reaction mixture to 1mL with CH₃CN. After 60 minutes the conversion (GC-FID) is 99.5% with selectivity (GC-FID) 99.5%. The final product **(2)** is then isolated via column chromatography with isolated yield of 99%.

### - Oxidation of Diphenyl sulfide to (3)

The reactant Diphenyl sulfide (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 90 minutes, the conversion (GC-FID) is >99% with selectivity (GC-FID) of >99%. The final product **(3)** is then isolated via column chromatography with isolated yield of 91%. When the methodology is applied on Diphenyl sulfide (0.6 mmol) dissolved in 10 mL of CH₃CN (80%) and H₂O (20%), in 60 minutes the conversion (GC-FID) is 85% with selectivity (GC-FID) of 99%.

### - Oxidation of Phenyl cyclopropyl sulfide to (4)

The reactant Phenyl cyclopropyl sulfide (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1 atm). Reaction is followed via GC FID diluting 100uL of reaction mixture to 1mL with CH₃CN. In 120 minutes, the conversion (GC-FID) is 85% with selectivity (GC-FID) of >99%. The final product **(4)** is then isolated via column chromatography with isolated yield of 82%.

### - Oxidation of p-Hydroxymethylthioanisole to (5)

The reactant p-Hydroxymethylthioanisole (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 30 minutes, the conversion (GC-FID) is >99%. The final product **(5)** is then isolated via column chromatography with an isolated yield of 86%.

### - Oxidation of p-Acetylthioanisole to (6)

The reactant p- Acetylthioanisole (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 7 minutes, the conversion (GC-FID) is >99%. The final product **(6)** is then isolated via column chromatography with an isolated yield of 84%.

### - Oxidation of 4-Methoxythioanisole to (7)

The reactant 4-Methoxythioanisole (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). The final product **(7)** is isolated after 50 minutes of reaction via column chromatography with isolated yield of 80%.

### - Oxidation of Para methyl thioanisole to (8)

The reactant Para methyl thioanisole (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 150 minutes, the conversion (GC-FID) is >99% with selectivity (GC-FID) >99%. The final product **(8)** is then isolated via column chromatography with isolated yield of 91%.

### - Oxidation of 2-(Methylthio)pyridine to (9)

The reactant 2-(Methylthio)pyridine (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 40 minutes, the conversion (GC-FID) is 99% with selectivity (GC-FID) of 99%. The final product **(9)** is then isolated via column chromatography with isolated yield of 70%.

### - Oxidation of 2-Methylthiobenzoxazole to (10)

The reactant 2-Methylthiobenzoxazole (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 90 minutes, the conversion (GC-FID) to **(10)** is 0%.

### - Oxidation of p-lodothioanisole to (11)

The reactant p-lodothioanisole (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 60 minutes, the conversion (GC-FID) to (11) is 8% with selectivity (GC-FID) of 84%.

### - Oxidation of p-Fluorothioanisole to (12)

The reactant p-Fluorothioanisole (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 120 minutes, the conversion (GC-FID) is >99% with selectivity (GC-FID) of 99%. The final product **(12)** is then isolated via column chromatography with Isolated yield of 66%.

### - Oxidation of p-Clorothioanisole to (13)

The reactant p-Clorothioanisole (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 30 minutes, the conversion (GC-FID) is >99% with selectivity (GC-FID) of 97%. The final product **(13)** is then isolated via column chromatography with isolated yield of 86%

### - Oxidation of [(Trifluoromethyl)thio]benzene to (14)

The reactant [(Trifluoromethyl)thio]benzene (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 90 minutes, the conversion (GC-FID) to **(14)** is 0%.

### - Oxidation of 3,5-dichloro thioanisole to (15)

The reactant 3,5-dichloro thioanisole (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 20 minutes, the conversion (GC-FID) to **(15)** is 98% with selectivity (GC-FID) of 96%. The final product is then isolated via column chromatography with isolated yield of 89%.

### - Oxidation of Tetrahydrothiophene to (16)

The reactant Tetrahydrothiophene (1 mmol) was dissolved in 10 mL of CH₃CN (80%) and H₂O (20%) with the presence of Toluene (2mmol) and LiCI (1mmol). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1 mL with CH₃CN. In 150 minutes, the conversion (GC-FID) is >99% with selectivity (GC-FID) >99%. The final product **(16)** is then isolated via column chromatography with isolated yield of 85.5%.

### - Oxidation of 1 ,4-Oxathiane to (17)

The reactant 1 ,4-Oxathiane (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 60 minutes, the conversion (GC-FID) to **(17)** is 0%.

### - Oxidation of 4-Hydroxytetrahydrothiopyran to (18)

The reactant 4-Hydroxytetrahydrothiopyran (1 mmol) was dissolved in 5 mL of CH₃CN (80%) and H₂O (20%) with Toluene as additives(2mmol). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 300 minutes, the conversion (GC-FID) is 100%. Product **(18)** is extracted using Water/DCM with a yield of 92%

### - Oxidation of Triphenylphosphine to (19)

The reactant Triphenylphosphine (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 15 minutes, the conversion (GC-FID) is >99% with selectivity (GC-FID) of >99%. The final product **(19)** is then isolated via column chromatography with isolated yield: 92,5%.

### - Oxidation of Triisopropyl phosphite to (20)

The reactant Triisopropyl phosphite (1 mmol) was dissolved in 10 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via UPLC-MS diluting 100uL of reaction mixture to 1mL with CH₃CN. In 15 minutes, the conversion (UPLC-MS) is >99%. The final product **(20)** is then isolated via column chromatography with isolated yield of 65%.

### - Oxidation of Trimethyl phosphite to (21)

The reactant Trimethyl phosphite (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 60 minutes, the conversion (GC-FID) to **(21)** is 56%.

### - Oxidation of Diphenyl Selenide to (22)

The reactant Diphenyl Selenide (1 mmol) was dissolved in 10 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via UPLC-MS diluting 100uL of reaction mixture to 1mL with CH₃CN. In 180 minutes, the conversion (UPLC-MS) to **(22)** is >99%. The final product is then isolated via column chromatography with isolated yield of 67%.

### - Oxidation of Bis(2-Diphenylphosphinophenyl)ether to (23)

The reactant Bis(2-Diphenylphosphinophenyl)ether (1 mmol) was dissolved in 10 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via UPLC-MS diluting 100uL of reaction mixture to 1mL with CH₃CN. In 40 minutes, the conversion (UPLC-MS) is >99. The final product **(23)** is then isolated via column chromatography with isolated yield of 61%.

### - Oxidation of Albendazole to (24)

The reactant Albendazole (1 mmol) was dissolved in 10 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 100 minutes, the conversion (GC-FID) is >99% with selectivity (GC-FID) of >99%. The final product **(24)** is then isolated via column chromatography with isolated yield of 85%.

### - Oxidation of Benzyl Methyl Sulfide to (25)

The reactant Benzyl Methyl Sulfide (0.6 mmol) was dissolved in 10 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 15 minutes, the conversion (GC-FID) to **(25)** is 98% with selectivity (GC-FID) of 32%. In 100 minutes, the conversion (GC-FID) to **(25)** is 98% with selectivity (GC-FID) of 35%.

### - 4-iodotetrahydrothiopyran

The reactant 4-iodotetrahydrothiopyran (1 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 60 minutes, the conversion (GC-FID) is 0%.

### - Thiophenol destruction

The reactant Thiophenol (0.6 mmol) was dissolved in 10 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 30 minutes, the conversion (GC-FID) is 100%.

### - Benzyl mercaptane destruction

The reactant Benzyl mercaptane (0.6 mmol) was dissolved in 10 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 60 minutes, the conversion (GC-FID) is 100%.

### - Oxidation of N,N-Dimethyldodecylamine

The reactant N,N-Dimethyldodecylamine (0.6mmol) was diluted in a 10 mL volumetric flask using a mixture of CH₃CN (80%) and H₂O (20%). The solution is then transferred in a 20mL test tube and stirred with a magnetic stirrer. The reaction tube is irradiated using a violet LED (peak wavelength 365nm), at room temperature, bubbling oxygen trough the solution (1atm). Reaction is followed via UPLC-MS and UPLC- diluting 100uL of reaction mixture to 1mL with CH₃CN. In 60 minutes the conversion was 0%.

### - Oxidation of Pyridine

The reactant Pyridine (0.6mmol) was diluted in a 10 mL volumetric flask using a mixture of CH₃CN (80%) and H₂O (20%). The solution is then transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. In 60 minutes the conversion was 0%.

The compounds obtained by means of the oxidation reaction described in the present application, the reaction time and the respective yields are showed here below.

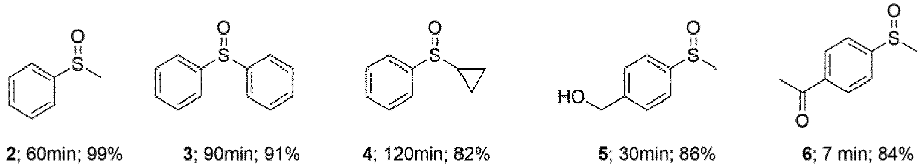

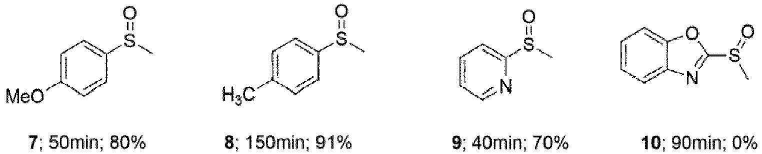

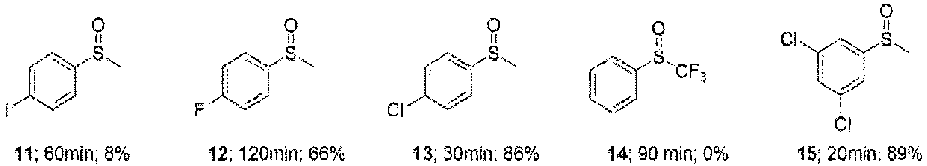

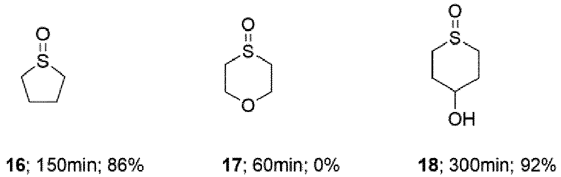

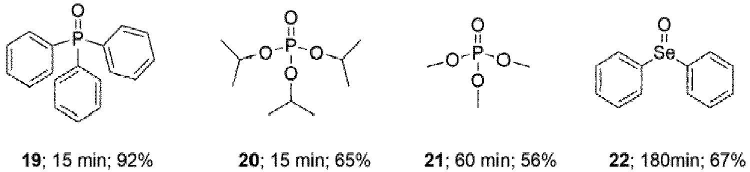

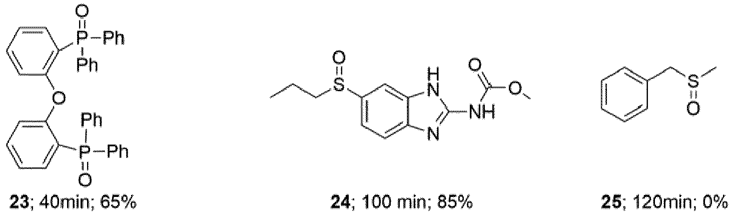

### EFFECT OF TEMPERATURE

The reactant Triphenylphosphine (7.5 mmol) was diluted in a 100 mL volumetric flask using CH₃CN (80%). The solution containing the reactant pumped by pump A at 4ml/min was mixed in a Y mixing element to demineralized water pumped by pump B at 1ml/min. The mixture is then mixed in a 2 mL loop and heated at 60°C. The warm solution is then pulsated by a pulsator and mixed with O₂ before entering the reactor HANU 2x5. Between the 2mL loop and the pulsator a back pressure valve of 100 psi is inserted. The reactor is irradiated using a violet LED (peak wavelength 365nm) lamp and thermostat at different temperatures. After the reactor a back pressure regulator set at 90 psi was used. On the gas stream a 75 psi back pressure regulator is used.

| Temperature | Conversion |
|---|---|
| 5°C | 39% |
| 20°C | 69% |
| 40°C | 88% |
| 60°C | 97% |

### SOLVENT SCOPE

The studies regarding the solvent have been performed using thioanisole as model substrate. For all the reactions the reactant (0,6 mmol) was dissolved. The solution is transferred in a 20mL test tube and stirred with a magnetic stirrer. The reaction tube is irradiated using a violet LED (peak wavelength 365nm), at room temperature, bubbling oxygen trough the solution (1atm) except otherwise specified. Reaction is followed via GC FID diluting 100uL of reaction mixture to 1mL with CH₃CN. Conversion and selectivity are measured via GC-FID. Solvents tried include acetonitrile (CH₃CN), water (H₂O), ethanol (EtOH), methanol (MeOH), 2-Methyltetrahydrofuran (2MeTHF), γ-Valerolactone (GVL), decane, toluene and combinations thereof.

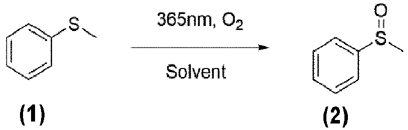

**Table 1**

| **Entry** | **Solvent** | **Time(min)** | **Conversion(%)** | **Selectivity(%)** |
|---|---|---|---|---|
| 1 | CH₃CN | 60 | 47 | 97 |
| 2 | CH₃CN/H₂O (4/1) | 60 | >99 | >99 |
| 3 | CH₃CN/H₂O (9/1) | 60 | 90 | >99 |
| 4 | CH₃CN/H₂O (1/4) | 60 | 98 | 99 |
| 5 | H₂O | 40 | 97 | 98 |
| 6 | EtOH/H₂O (4/1) | 60 | 55 | 100 |
| 7 | MeOH | 60 | 31 | >99 |
| 8 | MeOH/H₂O (4/1) | 60 | 61 | >99 |
| 9 | 2MeTHF | 60 | 51 | 14 |
| 10 | 2MeTHF/H₂O (4/1) | 60 | 60 | 21 |
| 11 | GVL | 60 | 13 | >99 |
| 12 | GVL/H₂O (4/1) | 60 | 53 | >99 |
| 13 | Decane | 60 | 26 | 86 |
| | | 40 | 3 | 100 |
| 14 | Decane/H₂O (4/1) | 60 | 6 | >99 |
| 15 | Toluene | 40 | 100 | 11 |
| 16^{a} | CH₃CN/H₂O (4/1) | 10 | 4 | 100 |
| | | 60 | 4 | 100 |
| 17^{b} | CH₃CN/H₂O (4/1) | 60 | 98 | >99 |
| 18^{c} | CH₃CN/H₂O (4/1) | 60 | 99 | >99 |

The results of the studies on the influence of the solvent on the reaction are illustrated in Table 1. The results in Table 1 are obtained with a reaction performed with (1) (0.6mmol) in a total volume of solvent of 10 mL bubbling oxygen using a balloon (1 bar) a) Saturated with O₂ and then no more oxygen is provided b) bubbling air c) open vessel.

### WAVELENGHT CUT OFF

The studies regarding the light source have been performed using thioanisole as model substrate. For all the reactions the reactant (0,6 mmol) was dissolved in 10 mL of CH₃CN (80%) and H₂O (20%). The solution is transferred in a 20mL test tube and stirred with a magnetic stirrer. The reaction tube is irradiated using the specified light source, at room temperature, bubbling oxygen trough the solution (1atm). Reaction is followed via GC FID diluting 100uL of reaction mixture to 1mL with CH₃CN. Conversion and selectivity are measured via GC-FID

**Table 2**

| **Entry** | **Light source** | **Time** | **Conversion(%)** | **Selectivity(%)** |
|---|---|---|---|---|
| 1 | 365nm | 60 | >99 | >99 |
| 2 | 405nm | 120 | 0 | 0 |
| 3 | 420nm | 120 | 0 | 0 |
| 4^{a} | Dark | 120 | 0 | 0 |

The results of the studies on the influence of the wavelength cut off on the reaction are illustrated in Table 2. The results in Table 2 are obtained with a reaction performed with **(1)** (0.6mmol) in a total volume of solvent of 10 mL 8:2 CH₃CN:H₂O a) solution in water bath at 40°C.

### Oxidation of Triphenylphosphine to (19) at 405nm

After the possibility to use 405 nm LED sources for triphenylphosphine (1 mmol) was dissolved in 5 mL of CH₃CN (80%) and H₂O (20%). The solution is transferred in a 20mL test tube and stirred with a magnetic stirrer. The reaction tube is irradiated using a blue LED (peak wavelength 405nm), at room temperature, bubbling oxygen trough the solution (1atm). Reaction is followed via GC FID diluting 100uL of reaction mixture to 1 mL with CH₃CN. In 120 minutes, the conversion (GC-FID) is 27%.

### ADDITIVES

### - Aromatic Additives

For molecules not containing aromatic moieties the addition of an additive in quantities between 1 and 2 equivalent can be beneficial, while higher concentrations led to lower selectivity.

### • Tetrahydrothiophene

The reactant tetrahydrothiophene (0,6 mmol) was dissolved in 10 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1atm). Reaction is followed via GC FID diluting 100uL of reaction mixture to 1mL with CH₃CN. Conversion and selectivity are measured via GC-FID. The same procedure is repeated with the addition of 1 equivalent of Toluene (entry 2) and using a mixture of toluene (80%) water (20%) as medium (entry 3). Electro donating and withdrawing effect on the additive's ring is tested using 1 equivalent of Anisole (entry 4), 1 equivalent of Triflorotoluene (entry 5) and a mixture of Anisole (80%) water (20%) as medium (entry 6).

| **Entry** | **Variations** | **Time** | **Conversion(%)** | **Selectivity(%)** |
|---|---|---|---|---|
| 1 | no | 60 | 7 | 100 |
| 2 | 1 eq Toluene | 60 | 37 | >99 |
| | | 120 | 99 | 100 |
| 3 | Toluene:Water 80:20 | 30 | >99 | 30 |
| 4 | 1 eq Anisole | 120 | >99 | >99 |
| 5 | 1 eq Trifluorotoluene | 120 | 70 | >99 |
| 6 | Anisole:Water 80:20 | 20 | 97 | - |

### • 4-Hydroxytetrahydrothiopyran

The reactant 4-Hydroxytetrahydrothiopyran (0,6 mmol) was dissolved in 5 mL of CH₃CN (80%) and H₂O (20%). The solution is transferred in a 20mL test tube and stirred with a magnetic stirrer. The reaction tube is irradiated using a violet LED (peak wavelength 365nm), at room temperature, bubbling oxygen trough the solution (1 atm). Reaction is followed via GC FID diluting 100uL of reaction mixture to 1 mL with CH₃CN. Conversion and selectivity are measured via GC-FID.

| **Entry** | **Variations** | **Time** | **Conversion(%)** | **Selectivity(%)** |
|---|---|---|---|---|
| 1 | no | 60 | 6 | >99 |
| 2 | 1 eq Toluene | 30 | 16 | >99 |

### - Salts

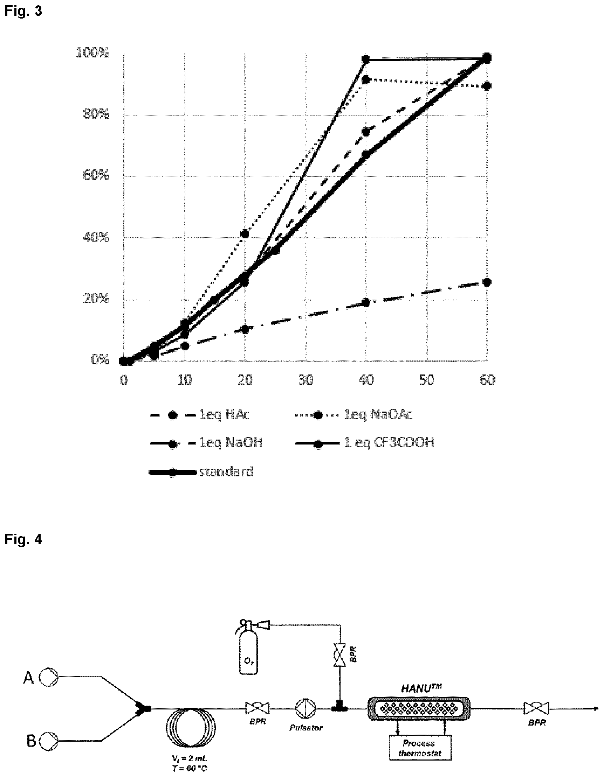

**Fig. 3** illustrates the results of studies regarding the addition of salts to the reaction medium. The studies regarding the addition of salt additives have been performed using thioanisole as model substrate. For all the reactions the reactant (0,6 mmol) was dissolved in 10 mL of CH₃CN (80%) and H₂O (20%) and 1 equivalent of salt (0.6mmol) was added. The solution is transferred in a 20mL test tube and stirred with a magnetic stirrer. The reaction tube is irradiated using a violet LED (peak wavelength 365nm), at room temperature, bubbling oxygen trough the solution (1atm. Reaction is followed via GC FID diluting 100uL of reaction mixture to 1mL with CH₃CN. In the figure the amount of sulfoxide is graphed against time. Salts tried as additives in the present example include LiBr, NaCl, LiCI, KBr, LiF and Lil.

| **Entry** | **Variations** | **Time** | **Conversion(%)** | **Selectivity(%)** |
|---|---|---|---|---|
| 1 | No | 60 | 99 | 99 |
| 2 | LiBr | 40 | 97 | 99 |
| 3 | NaCl | 20 | 98 | 99 |
| 4 | LiCl | 20 | 91 | 99 |
| 5 | KBr | 60 | 97 | 99 |
| 6 | LiF | 40 | 98 | 99 |
| 7 | Lil | 40 | 0 | |

### - Acid-Base

**Fig. 3** illustrates the results of studies regarding the addition of acids or bases to the reaction medium. The studies have been performed using thioanisole as model substrate. For all the reactions the reactant (0,6 mmol) was dissolved in 10 mL of CH₃CN (80%) and H₂O (20%) and 1 equivalent of additive (0.6mmol) was added. After addition of the additive, the solution is transferred in a 20mL test tube and stirred with a magnetic stirrer. The reaction tube is irradiated using a violet LED (peak wavelength 365nm), at room temperature, bubbling oxygen trough the solution (1atm. Reaction is followed via GC FID diluting 100uL of reaction mixture to 1mL with CH₃CN. In **Error! Reference source not found**.the figure the amount of sulfoxide is graphed against time. Various acids and bases were used, in particular, acetic acid (HAc), sodium acetate (NaOAc), sodium hydroxide (NaOH), trifluoroacetic acid (CF₃COOH).

| **Entry** | **Variations** | **Time** | **Conversion(%)** | **Selectivity(%)** |
|---|---|---|---|---|
| 1 | no | 60 | 99 | 99 |
| 2 | HAc | 60 | 99 | 99 |
| 3 | NaOAc | 40 | 99 | 91 |
| 4 | NaOH | 60 | 32 | 80 |
| 5 | CF3COOH | 40 | 99 | 99 |

### FLOW EXPERIMENTS

The set up shown in **Fig. 4** illustrates how a set-up for the flow oxidation of species according to the method of the present invention can be conducted.

### - Flow Oxidation of PPh₃

The reactant Triphenylphosphirine (7.5 mmol) was diluted in a 100 mL volumetric flask using CH₃CN (80%). The solution containing the reactant pumped by pump A with a flow rate of 1ml/min was mixed in a Y mixing element to demineralized water pumped by pump B with a flow rate of 0.25ml/min. The mixture is then mixed in a 2 mL loop and heated at 60°C. The warm solution is then pulsated by a pulsator and mixed with O₂ before entering the reactor HANU 2x5. Between the 2mL loop and the pulsator a back pressure valve of 100 psi is inserted. The reactor is irradiated using a violet LED (peak wavelength 365nm) lamp and thermostat at 60°C. After the reactor a back pressure regulator set at 90 psi was used. On the gas stream a 75 psi back pressure regulator is used. The final product is dried and filtered using cold acetone yielding 75% product.

### - Flow Oxidation of Thioanisole

A solution containing the reactant Thioanisole (0.06M) In a solution of CH₃CN 80% H2O 20% is prepared. The solution is then pumped in a continuous reactor HANU 5 using HPLC pump with a flow rate of 0.5mL/min. After the pump the solution went through a pulsator to mix the oxygen and the solution. The liquid flow is mixed with a oxygen flow via a y junction just before the HANU 5 reactor. The reactor is irradiated using a violet LED (peak wavelength 365nm), at room temperature. A 7 Bar back pressure regulator is used. In 10 minutes residence time the conversion to the product was 20%, with a selectivity of 100%.

### - Effect of residence time on flow oxidation of PPh₃

The reactant Triphenylphosphine (7.5 mmol) was diluted in a 100 mL volumetric flask using CH₃CN (80%). The solution containing the reactant pumped by pump A was mixed in a Y mixing element to demineralized water pumped by pump B. The mixture is then mixed in a 2 mL loop and heated at 60°C. The warm solution is then pulsated by a pulsator and mixed with O₂ before entering the reactor HANU 2x5. Between the 2mL loop and the pulsator a back pressure valve of 100 psi is inserted. The reactor is irradiated using a violet LED (peak wavelength 365nm) lamp and thermostat at 20°C. After the reactor a back pressure regulator set at 90 psi was used. On the gas stream a 75 psi back pressure regulator is used.

| Flow rate pump A | Flow rate Pump B | Conversion |
|---|---|---|
| 0.2 ml/min | 0.8 ml/min | > 99% |
| 0.4 ml/min | 1.6 ml/min | 98% |
| 1 ml/min | 4 ml/min | 69% |
| 2 ml/min | 8 ml/min | 39% |

### REFERENCES

1. Rajabi, Fatemeh, et al. "Highly efficient and selective aqueous aerobic oxidation of sulfides to sulfoxides or sulfones catalyzed by tungstate-functionalized nanomaterial." Molecular Catalysis 515 (2021): 111931.
2. Bonesi, Sergio M., et al. "Direct irradiaton of aryl sulfides: Homolytic fragmentation and sensitized S-oxidation." The Journal of Organic Chemistry 82.17 (2017): 9054-9065.
3. Fan, Qiangwen, et al. "Catalyst-free visible light-mediated selective oxidation of sulfides into sulfoxides under clean conditions." Green Chemistry 23.20 (2021): 7945-7949.

## Claims

1. A method of oxidation of an organic compound comprising a heteroatom selected from S, Se, P, N, the method comprising the steps of:
a) providing an organic compound comprising a heteroatom selected from S, Se, P, N, in a reaction medium comprising H₂O, and optionally a reaction additive;
b) irradiating in the presence of a source of oxygen the reaction medium comprising the compound provided at step a) with light having a wavelength in the UV-vis electromagnetic spectrum, thereby oxidating said heteroatom.

2. The method according to the previous claim, wherein step b) is conducted in the presence of a compound comprising at least one aromatic moiety.

3. The method according to the previous claim, wherein the aromatic moiety is selected from: phenyl, naphthalene, pyridine, each either substituted or unsubstituted.

4. The method according to the previous claim, wherein the aromatic moiety is a phenyl ring, either substituted or unsubstituted.

5. The method according to any one of the previous claims, wherein the reaction medium is H₂O.

6. The method according to one of the previous claims, wherein at step a) the reaction medium further comprises a polar solvent.

7. The method according to the previous claim, wherein the polar solvent is acetonitrile.

8. The method according to the previous claim, wherein the reaction medium comprises H₂O and acetonitrile in a ratio HzO:acetonitrile from 100:0 to 1:99, preferably 20:80.

9. The method according to any one of the previous claims, wherein the reaction additive is a compound selected from the list comprising: toluene, benzene, xylene, anisole, trifluorotoluene.

10. The method according to any one of claims 1 to 8, wherein the reaction additive is a salt selected from: LiBr, NaCl, LiCI, KBr, LiF, and is preferably LiCl.

11. The method according to any one of claims 1 to 8, wherein at step a) the reaction additive is a Bronsted acid, such as acetic acid, trifluoroacetic acid.

12. The method according to any one of the previous claims, wherein at step a) the heteroatom is S.

13. The method according to any one of the previous claims, wherein at step a) the heteroatom is P.

14. The method according to any one of the previous claims, wherein at step a) the heteroatoms is Se.

15. The method according to any one of the previous claims, wherein at step a) the heteroatoms is N.

16. The method according to any one of the previous claims, wherein the source of oxygen is a mixture comprising nitrogen and oxygen, such as air.

17. The method according to any one of the previous claims, wherein at step b) light is with a wavelength from 320nm, preferably from 320nm to 400nm.

18. The method according to any one of the previous claims, wherein the method is part of a desulfurization treatment.

19. The method according to any one of the previous claims, wherein steps a) and b) are carried out as a continuous flow oxidation.
